# EUROPÄISCHE PATENTSCHRIFT

(11) **EP 2 967 971 B1**
(45) Veröffentlichungstag und Bekanntmachung des Hinweises auf die Patenterteilung: **21.02.2018**
(21) Anmeldenummer: 14709286.0
(22) Anmeldetag: 11.03.2014
(51) Int. Cl.: A61F 5/56

(54) **VERFAHREN ZUM HERSTELLEN EINER PROTRUSIONSSCHIENE**
METHOD FOR PRODUCING A PROTRUSION SPLINT
PROCÉDÉ DE FABRICATION D'UN PROPULSEUR MANDIBULAIRE

(30) Priorität: 12.03.2013 DE 102013102473
(43) Veröffentlichungstag der Anmeldung: 20.01.2016
(73) Patentinhaber: Hicat GmbH, 53177 Bonn (DE)
(72) Erfinder: GRÜNBERG, Daniel, 53179 Bonn (DE)
(74) Vertreter: Braun-Dullaeus Pannen Patent- und Rechtsanwälte
(86) Internationale Anmeldenummer: PCT/EP2014/054695
(87) Internationale Veröffentlichungsnummer: WO 2014/140007

(56) Entgegenhaltungen:
- WO-A1-2012/172112
- US-A- 5 921 942
- US-A1- 2007 209 666
- US-A1- 2007 255 161
- US-A1- 2010 316 973

## Beschreibung

Die Erfindung betrifft ein Verfahren zum Herstellen einer Protrusionsschiene.

Protrusionsschienen zur Behandlung von Schnarchen sind nach dem Stand der Technik bereits in unterschiedlichen Ausführungsformen bekannt. Es handelt sich hierbei um im herausnehmbare Zahnschienen z.B. aus Kunststoff, die kieferorthopädischen Apparaturen oder sog. Knirscherschienen ähneln. Diese werden während des Schlafes im Mund getragen werden und beeinflussen die Unterkieferlage, die Zungenlage sowie andere Weichteile. Dabei werden diese Körperteile in einer vorderen Position (= Protrusion) halten (u. a. bekannt durch US 5,462,066 A und EP 1 203 570 B1). Dadurch wird insbesondere bei Rückenlage des Patienten ein Zurückfallen des Unterkiefers und der anhängenden Weichteile verhindert. Es resultiert daraus eine Vergrößerung des Rachenraumes mit freiem Atemweg während des Schlafes und eine gesunde nächtliche Atmung ohne Atemaussetzer und ohne Schnarchen.

Die bekannten Verfahren zur Herstellung der Protrusionsschiene nutzen zur Einstellung der Stellung der Protrusion hauptsächlich Erfahrungswerte, die durch Versuche im Schlaflabor verifiziert werden. Es erfolgt zunächst eine Einstellung der mutmaßlich geeigneten Stellung; dann muss der Patient ein oder mehrere Nächte mit eingesetzter Protrusionsschiene im Schlaflabor verbringen, um zu überprüfen, ob die Behandlung mit der entsprechend eingestellten Protrusionsschiene den gewünschten Erfolg liefert. Diese Versuche sind jedoch aufwendig und für den Patienten wenig komfortabel. Ferner hat es sich herausgestellt, dass die langfristige Anwendung der Protrusionsschiene sich nachteilig auf die Kiefergelenke auswirken kann. So können durch die Beaufschlagung der Kiefer in die durch die Schiene vorgegebene Stellung die Kiefergelenke in einen Zustand gebracht werden, der unnatürlich ist, und so die Kiefergelenke beschädigen kann. Dies führt dazu, dass an Patienten mit Kiefergelenksproblemen die Behandlung mittels Protrusionsschiene grundsätzlich nicht durchgeführt wird.

Aus der US 2010/0316973 A1 ist ein Verfahren zum Herstellen einer Protrusionsschiene bekannt, bei dem der Unterkieferbereich gegenüber dem Oberkieferbereich nach vorne verlagert in einer ersten Stellung fixiert wird. In dieser Stellung wird der Sitz kontrolliert. Die Protrusionsschiene wird nach eine eventuellen Korrektur der Position angefertigt.

Aus der US 2007/0255161 A1 sind Mesungen an Patienten mit Schlaf Apnoe zur Modellierung des Durchgangs der Luftröhre bekannt.

Es ist daher die Aufgabe, verbessertes Verfahren zum Herstellen einer Protrusionsschiene bereitzustellen. Die der Erfindung zugrunde liegende Aufgabe wird gelöst durch ein Verfahren nach Anspruch 1; bevorzugte Ausgestaltungen ergeben sich aus den Unteransprüchen.

Der Kern der Erfindung liegt insbesondere darin, dass zur Kontrolle der ersten Stellung die Erkenntnisse herangezogen werden, die anhand des dreidimensionalen Bilddatensatzes gewonnen wurden. Der Bilddatensatz wurde insbesondere mit einem tomografischen Bildgebungsverfahren erstellt. Der Kiefer wird in der ersten Stellung insbesondere durch eine verstellbare Protrusionsschiene gehalten. Die erste Stellung ist eine solche Stellung, bei der der Kiefer aus seiner Normalstellung derart herausgeschoben wird, so dass der Unterkiefer bezogen auf die Normalstellung ein Stück nach vorne (anterior) verlagert ist. Die erste Stellung kann bereits die zweite Stellung darstellen, sofern sich ergibt, dass ein Behandlungserfolg durch diese Stellung gewährleistet ist und insbesondere keine Schäden an den Kiefergelenken erzeugt werden. So wird in der ersten Stellung zunächst überprüft, ob der entsprechende Luftdurchgang so vergrößert wurde, dass mit einer Verringerung des Schnarchens zu rechnen ist. Dafür kann der dreidimensionale Bilddatensatzes derart ausgewertet werden, dass eine Öffnungsgröße des Luftdurchgangs ermittelt wird. Eine solche Öffnungsgröße kann beispielsweise der kleinste Querschnitt der Luftröhre während der ersten Stellung sein; es kann allerdings auch das Gesamtvolumen eines Abschnitts der Luftröhre im zu beobachtenden Bereich der Luftröhre sein, was also einem Aufintegrieren der einzelnen Öffnungsquerschnitte über die Länge des zu beobachtenden Luftröhrenabschnitts entspricht. Ferner kann grundsätzlich die Form des Luftdurchgangs überprüft werden oder ein Abstand zweier gegenüberliegender Luftröhrenwandungen zueinander.

Anhand der ermittelten Öffnungsgröße kann erkannt werden, ob die eingenommene erste Stellung geeignet ist, einen Behandlungserfolg zu bewirken. Hierzu wird bevorzugt ein Vergleich der ermittelten Öffnungsgröße mit einer Zielvorgabe durchgeführt. Die Zielvorgabe kann ein Wert oder Wertebereich sein, in dem die Öffnungsgröße liegen muss.

Falls die Öffnungsgröße der Zielvorgabe entspricht, wird definiert, dass die erste Stellung zugleich die zweite Stellung darstellt. Falls ermittelt wird, dass die Öffnungsgröße der Zielvorgabe nicht entspricht, wird eine andere zweite Stellung ermittelt. Für den letztgenannten Fall kann die zweite Stellung auch rechnerisch ermittelt werden anhand einer Differenz der ermittelten Öffnungsgröße von der Zielvorgabe. Hierfür kann ein Korrekturwert ermittelt werden, mit dessen Hilfe die zweite Stellung aus der ersten Stellung berechnet wird. So kann der Korrekturwert beispielsweise einen Vorschubwert darstellen, um den der Unterkiefer gegenüber der ersten Stellung zu verlagern ist, damit dieser in eine geeignete zweite Stellung gelangt. Ein solcher Korrekturwert kann beispielsweise auch aus historischen Versuchsdaten abgeleitet werden, aus denen z.B. bekannt ist, dass ein Vorschub um den Wert X oder um x% eine Vergrößerung der Öffnungsgröße um den Wert Y oder um y% zur Folge hat. Ein solches rechnerisches Vorgehen ist insbesondere dann bevorzugt, wenn die Differenz der ermittelten Öffnungsgröße von der Zielvorgabe recht gering ist, zumal dann ein erneutes teures Erstellen des dreidimensionalen Bilddatensatzes vermieden wird. Andernfalls wird eine geänderte erste Stellung eingenommen; es wird dann erneut ein dreidimensionaler Bilddatensatz des Luftdurchgangs erstellt. Daraus wird dann wiederum die Öffnungsgröße des Luftdurchgangs in der geänderten ersten Stellung ermittelt und erneut entschieden, ob die geänderten erste Stellung als zweite Stellung definiert wird. Dieser Vorgang kann sich beliebig oft wiederholen.

Vorzugsweise wird für das Anfertigen der Protrusionsschiene ein Bissregistrat verwendet. Der Patient beißt auf das Bissregistrat, während sich seine Kiefer in einer der Stellungen befinden. Er hinterlässt dann Abdrücke von einigen Zähnen des Unterkiefers und des Oberkiefers, die zueinander entsprechend der Stellung ausgerichtet sind. Anhand des Bissregistrats können dann Abdrücke oder Scans der beiden Kiefer, die in einer beliebigen Stellung angefertigt wurden, entsprechend der eingenommenen Stellung zueinander ausgerichtet werden. Sind die Ausrichtungen der beiden Abdrücke oder Scans der Kiefer dann bekannt, so kann die Protrusionsschiene hergestellt werden, wie es beispielsweise in der DE 10 2009 009 916 A1 beschrieben ist.

Vorzugsweise wird zum Anfertigen der dreidimensionale Bilddatensatz mit Oberflächendaten eines optischen Scans der Kieferbereiche überlagert. Daraus werden dann Zahnnegative berechnet, auf deren Basis die Protrusionsschiene gefertigt wird. Oberflächenwerte, die allein aus dem dreidimensionalen Bilddatensatz extrahiert werden, sind üblicherweise nicht so exakt, dass diese allein für das Anfertigen der Protrusionsschiene verwendet werden können. Allerdings lässt sich aus dem dreidimensionalen Bilddatensatz sehr gut die relative Position der einzelnen Zähne zueinander ermitteln. Wenn nun die sehr guten Positionsdaten des dreidimensionalen Bilddatensatzes mit den sehr guten Oberflächendaten des optischen Scans verknüpft werden, so kann eine sehr exakte Protrusionsschiene ohne die Notwendigkeit eines Abdrucks angefertigt werden.

Das vorstehend beschriebene Verfahren ermöglicht es, dass die bestmögliche Stellung möglichst exakt getroffen wird. Dies ist insbesondere von Vorteil, da eine Stellung, bei der der Unterkiefer gegenüber der Normalstellung zu weit nach vorne verlagert wird, Probleme an den Kiefergelenken schaffen kann. Insofern ist es wünschenswert, den Unterkiefer zwar so weit wie nötig, aber möglichst wenig nach vorne zu verlagern.

Um die Probleme an den Kiefergelenken ferner zu vermeiden, ist es vorzugsweise vorgesehen, dass in der zweiten Stellung oder in der ersten Stellung der Zustand der Kiefergelenke untersucht wird. Dazu wird vorzugsweise ein dreidimensionaler Bilddatensatz der Kiefergelenke erstellt. Dieser dreidimensionale Bilddatensatz wird vorzugsweise gemeinsam mit dem dreidimensionalen Bilddatensatz des Luftdurchgangs in der ersten Stellung erstellt. Anhand dieser Bilddaten der Kiefergelenke kann dann festgestellt werden, ob durch das Verlagern in die Stellung Probleme an den Kiefergelenken hervorgerufen werden können. Gegebenenfalls kann dann die Protrusionsschiene noch optimiert werden, um die Probleme an den Kiefergelenken möglichst zu verhindern. Auch bei Personen, die bereits unter Kiefergelenksproblemen leiden, kann ermittelt werden, ob sich die Stellung nachteilig auf die bereits vorhandenen Kiefergelenksprobleme auswirkt. Falls nicht wird es durch das erfindungsgemäße Vorgehen ermöglicht, dass auch für Personen mit Kiefergelenksproblemen die Protrusionsschiene zur Behandlung des Schnarchens verwendet werden kann und nicht per se von vornherein ausgeschlossen wird.

Eine solche Protrusionsschiene eignet sich insbesondere für die Behandlung von störendem, nicht gesundheitsschädigendem Schnarchen. Grundsätzlich sind solche Schienen aber auch für weitere Anwendungen geeignet, insbesondere für die Behandlung von Schlafapnoe.

Die Erfindung wird anhand der Figuren nachfolgend näher erläutert. Hierin zeigt
- Figur 1: den Querschnitt eines Schädels
a) mit geringem Schnarchrisiko,
b) mit erhöhtem Schnarchrisiko;
- Figur 2: eine erfindungsgemäß hergestellte Protrusionsschiene;
- Figur 3: den Querschnitt eines Schädels mit erhöhtem Schnarchrisiko
a) mit eingesetzter Protrusionsschiene,
b) ohne eingesetzte Protrusionsschiene;
- Figur 4: schematisch ein Tomographiebild des Luftröhrenbereichs;
- Figur 5: schematisch unterschiedliche Querschnitte des Luftröhrenbereichs in Draufsicht bei Stellung des Kiefers
a) in geeigneter Stellung,
b) in nicht geeigneter Stellung,
c) in Normalstellung.

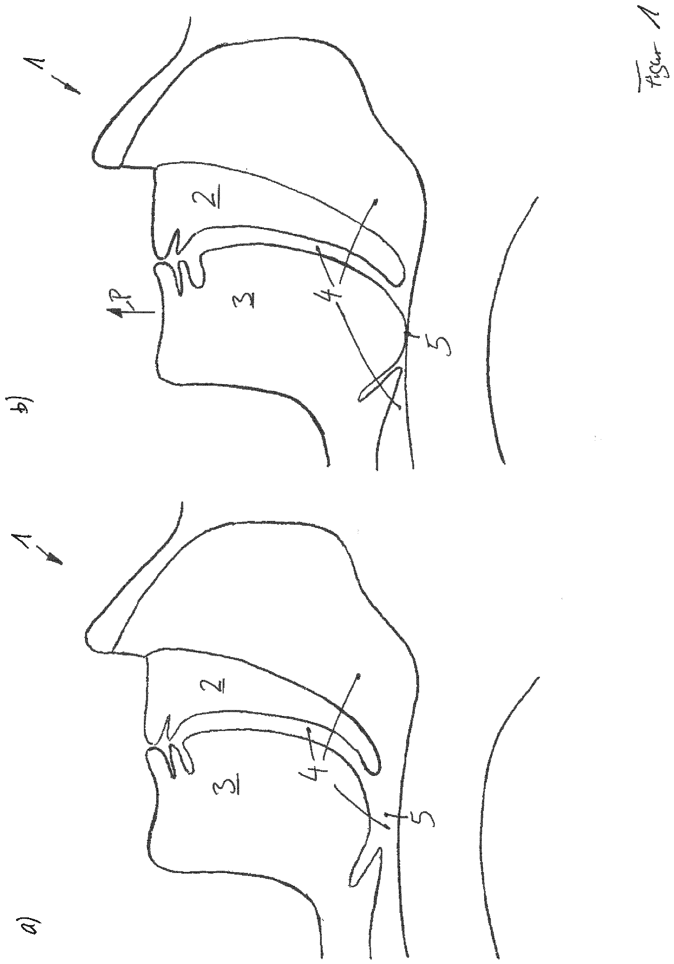

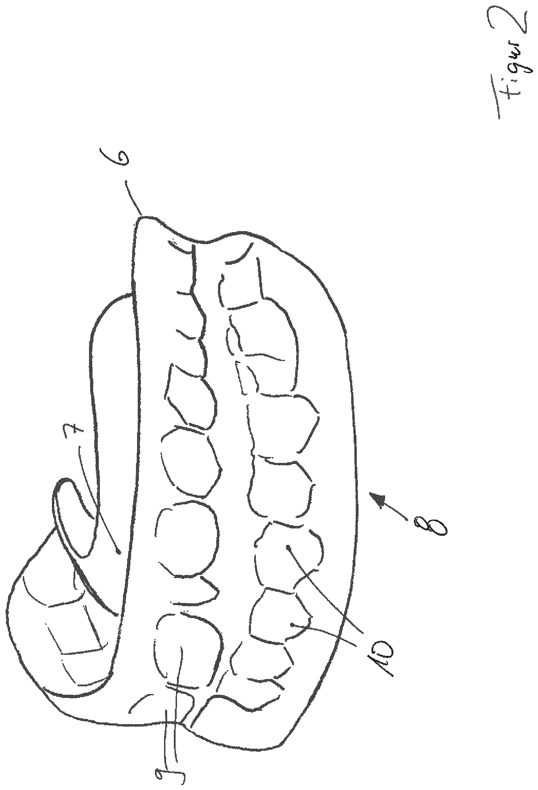

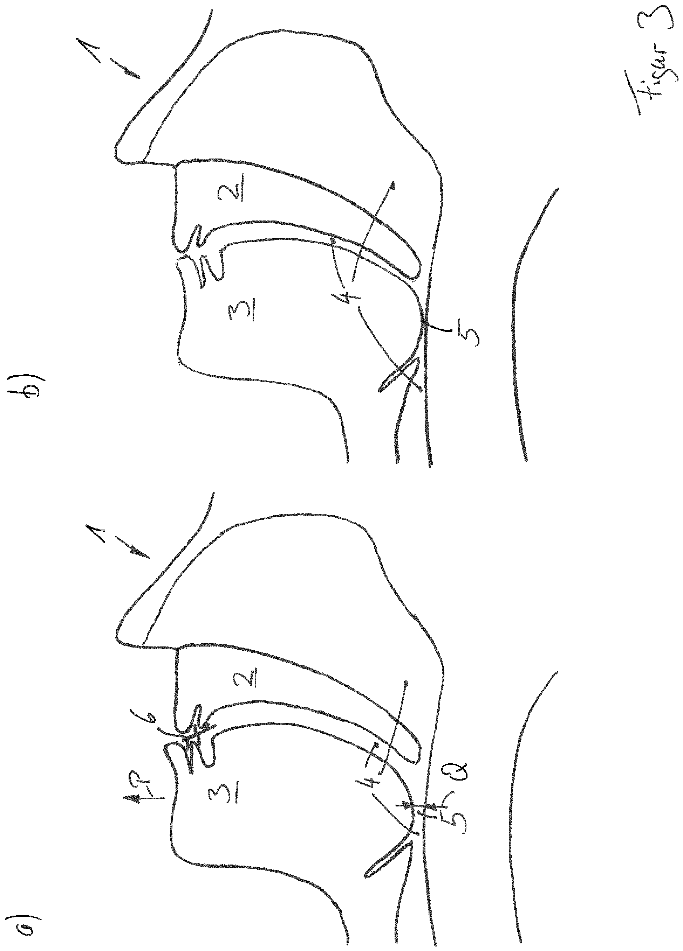

Figur 1 zeigt zwei Querschnittsdarstellungen eines menschlichen Schädels. Die Darstellung nach Figur 1a zeigt einen Schädel 1 mit geringem Schnarchrisiko. Am Schädel 1 sind der Oberkiefer 2 und der Unterkiefer 3 zu erkennen. Atemwege 4 im Bereich des Mundes, der Nasen und der Luftröhre sind eingezeichnet. Im Bereich einer Luftröhre ist ein Luftdurchgang 5 zu erkennen, der im ausreichend groß ausgebildet ist, um genügend Luft beim Schlafen in die Lunge und aus der Lunge strömen zu lassen. In der Darstellung nach Figur 1b, die den Querschnitt des menschlichen Schädels 1 mit erhöhten Schnarchrisikos zeigt, ist dieser Luftdurchgang 5 deutlich verkleinert gegenüber dem Zustand nach Figur 1a. Bedingt ist das Schnarchrisiko häufig durch eine Entspannung von Muskulaturen im Bereich der Atemwege. Eine häufig genutzte Möglichkeit zur Behandlung des Schnarchens ist das Verlagern des Unterkiefers in eine Protrusionsstellung gegenüber dem Oberkiefer, was in Figur 1b durch einen Pfeil P angedeutet ist. Gegenüber der Normalstellung der Kiefer 2, 3, wie sie in den Figuren 1a und 1b dargestellt ist, wird dann der Unterkiefer in eine Stellung gegenüber dem Oberkiefer gebracht, bei der also der Unterkiefer weiter nach vorne, also anterior, verlagert wird (siehe auch weiter unten Figur 3b). Hierzu wird eine Protrusionsschiene 6 verwendet, wie sie schematisch in Figur 2 gezeigt ist.

Die Protrusionsschiene 6 weist einen oberen Auflagebereich 7 auf, der eine Vielzahl von oberen Zahnnegativen 9 des Oberkiefers umfasst. Ebenso weist die Protrusionsschiene 6 einen unteren Auflagebereich 8 mit einer Vielzahl von Zahnnegativen 10 des Unterkiefers auf. Die oberen und unteren Zahnnegative 9, 10 wurden mithilfe eines Gipsabdrucks oder mithilfe eines digitalen Scans maschinell erzeugt. Die zu behandelnde Person kann nun mit seinem Oberkiefer 2 in den oberen Auflagebereich 7 beißen und mit dem Unterkiefer 3 in den unteren Auflagebereich 8 beißen. Wenn dann die Zähne des Oberkiefers 2 und des Unterkiefers 3 mit dem entsprechenden Zahnnegativen 9, 10 in Anlage sind, ist der Unterkiefer 2 gegenüber dem Oberkiefer 3 in der Stellung gehalten, die durch die Protrusionsschiene 6 vorgegeben wird. Im Rahmen der Erfindung werden dabei die Zähne als Bestandteil der Kiefer bzw. Kieferbereiche verstanden.

Diese Stellung wird ferner noch in Figur 3 visualisiert. Figur 3a zeigt dabei die Protrusionsschiene 6, die in Anlage nun mit dem Oberkieferbereich 2 und dem Unterkieferbereich 3 ist. Es ist zu erkennen, dass der Unterkieferbereich 3 gegenüber der Normalstellung (Figur 3b) nach vorne verschoben ist gemäß dem Pfeil P. Es stellt sich nun der Effekt ein, dass durch das "Nachvorneschieben" der Querschnitt Q des Luftdurchgangs 5 größer ist als im Normalzustand nach Figur 3b. Der Patient kann nun wieder gut atmen und hat einen ruhigen Schlaf.

Das Besondere ist nun, dass vor Anfertigung der Protrusionsschiene 6 nach Figur 2 die Kiefer 2, 3 in eine erste Stellung zueinandergebracht werden, die eine ähnliche oder auch identische Situation darstellt, wie sie in Figur 3a gezeigt ist. Es wird dann der Luftröhrenbereich einem bildgebenden dreidimensionalen Tomographieverfahren zum Erstellen eines dreidimensionalen Bilddatensatzes unterzogen, durch den detaillierte Erkenntnis über die räumlichen Zustände im Luftdurchgang 5 bereitstellt werden.

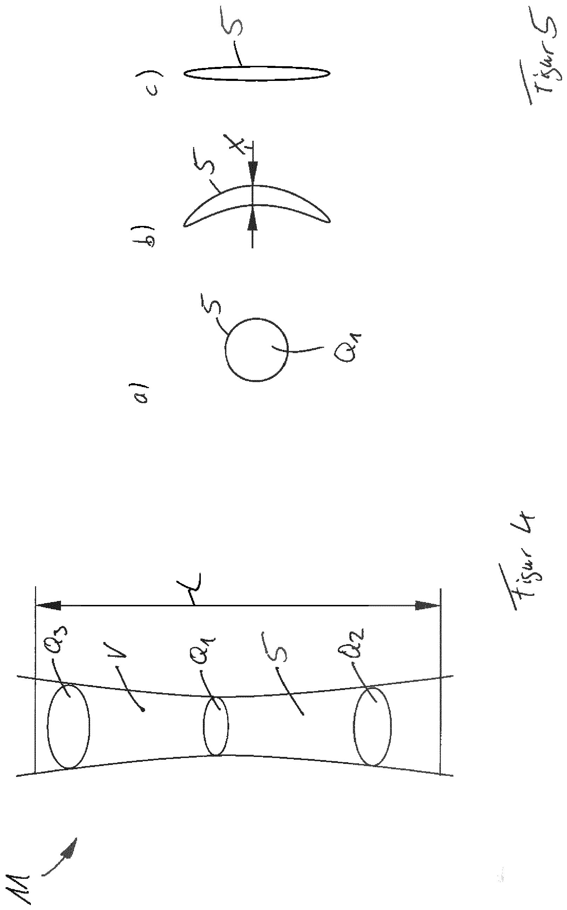

Figur 4 zeigt eine erstellte dreidimensionale Repräsentation 11 des Luftdurchgangs 5. Anhand dieser dreidimensionalen Repräsentation 11 können nun an unterschiedlichen Positionen des Luftdurchgangs die Querschnittsflächen Q₁, Q₂, Q₃ des Luftdurchgangs 5 berechnet werden. Eine Software, die solche Berechnungen durchführt, ist unter dem Namen "Dolphin Imaging 3D" erhältlich. So ist beispielsweise der Querschnitt Q₁ der kleinste Querschnitt im Luftdurchgang 5. Der kleinste Querschnittswert Q₁ kann eine maßgebliche Öffnungsgröße für die Beurteilung sein, ob der Luftdurchgang 5 für die Behandlung ausreichend ist. Dieser kleinste Querschnittswert Q₁ wird dann mit einer Zielvorgabe verglichen. Falls dieser Querschnittswert Q₁ dann größer ist als der Zielvorgabewert ist, so ist die erste Stellung dazu geeignet, den Luftdurchgang entsprechend so groß zu halten, so dass das Schnarchrisiko abgestellt werden kann. Die erste Stellung wird dann als zweite Stellung festgelegt. Sollte der Wert Q₁ allerdings kleiner als die Zielvorgabe sein, so wird festgestellt, dass die erste Stellung eben nicht geeignet ist, das Schnarchrisiko abzustellen. Es muss dann eine neue Stellung ermittelt werden, die ggf. über dieselben Schritte überprüft wird.

Aber auch ein zu großer Querschnitt Q₁ kann Aufschluss darüber geben, dass die erste Stellung nicht optimal ist. Denn ein zu großer Vorschub (Protrusion) kann zugleich eine recht unnatürliche Stellung erzeugen, welche zu weit entfernt ist von der Normalposition. Hieraus können Schäden an den Kiefergelenken resultieren. Daher ist es vorteilhaft, wenn neben den Untersuchungen des Luftdurchgangs auch eine Untersuchung der Kiefergelenke stattfindet. Dafür wird in dem vorher angesprochen bildgebenden Verfahren zugleich eine Aufnahme von den Kiefergelenken erstellt. Sollte sich ergeben, dass der Zustand des Kiefergelenk Schäden am Kiefergelenk hervorrufen kann, so wird die Stellung verändert.

Auch andere Größen, die durch die dreidimensionale Repräsentation ermittelbar sind, können für die Überprüfung der ersten Stellung herangezogen werden. Über eine Aufintegrierung der einzelnen Querschnitte Q₁, Q₂, Q₃, ... über eine Gesamtlänge L des Luftdurchganges 5 kann ein Gesamtvolumen V des Luftdurchganges 5 ermittelt werden. Auch dies kann die relevante Querschnittsgröße des Verfahrens sein und mit einem entsprechenden Zielvorgabewert verglichen werden.

In Figur 5 sind weitere mögliche Querschnitte gezeigt. Figur 5a zeigt einen kreisrunden Querschnitt Q₁ entsprechend Figur 4, der auf eine geeignete Stellung hindeutet. In Figur 5b ist eine Art u-förmige Querschnittsfläche Q₄ gezeigt. Zwar mag die Querschnittsfläche von ihrem Betrag ausreichend für eine erfolgreiche Behandlung sein; die Form der Querschnittsfläche ist allerdings ungünstig, da ein Abstand X zwischen zwei gegenüberliegenden Wandungen des Luftdurchgangs zu gering ist. Insofern stellt auch der Abstand X eine relevante Öffnungsgröße dar.

Für das Fixieren der Oberkiefer und Unterkiefer in der ersten Stellung kann eine einstellbare Protrusionsschiene verwendet werden. Diese weist getrennte obere und untere Auflagebereiche auf, die in unterschiedlichen Positionen zueinander gebracht werden können und darin fixiert werden können. Wenn der Patient dann diese einstellbare Protrusionsschiene trägt, wird der dreidimensionalen Bilddatensatz erstellt. Ferner wird das Bissregistrat hergestellt, während die zu behandelnde Person die einstellbare Protrusionsschiene trägt. Eine verstellbare Protrusionsschiene ist in der DE 20 2008 011 841 U1 gezeigt.

Die so hergestellte Protrusionsschiene ist auch für die Behandlung von Schlafapnoe einsetzbar.

### Bezugszeichenliste

- 1: Schädel
- 2: Oberkiefer
- 3: Unterkiefer
- 4: Atemwege
- 5: Luftdurchgang
- 6: Protrusionsschiene
- 7: oberer Auflagebereich
- 8: unterer Auflagebereich
- 9: Zahnnegativ (Oberkiefer)
- 10: Zahnnegativ (Unterkiefer)
- 11: dreidimensionale Repräsentation

- Q: Querschnittsfläche im Luftdurchgang
- L: Länge des Luftdurchgang
- V: Volumen des Luftdurchgang
- X: Abstand zweier gegenüberliegender Wandungen des Luftdurchganges

## Patentansprüche

1. Verfahren zum Herstellen einer Protrusionsschiene (6),
wobei die Protrusionsschiene (6) einen oberen Auflagebereich (7) zur definierten Auflage für einen Oberkieferbereich (2) und einen unteren Auflagebereich (8) zur definierten Auflage für einen Unterkieferbereich (3) umfasst, wobei bei Auflage der Kieferbereiche (2, 3) an den jeweiligen Auflagebereichen (7,8) der Unterkieferbereich (3) relativ zum Oberkieferbereich (2) in einer durch die Protrusionsschiene (6) vorgegebenen Stellung gehalten ist, umfassend die folgenden Verfahrensschritte:
**Fixieren** der Kieferbereiche (2,3) in einer ersten Stellung, wobei in der ersten Stellung der Unterkieferbereich (3) gegenüber dem Oberkieferbereich (2) nach vorne verlagert ist verglichen mit der Normalstellung der Kiefer,
**Erstellen** eines dreidimensionalen Bilddatensatzes (11) eines Luftdurchgangs (5) im Bereich der Luftröhre (5) während die Kieferbereiche (2, 3) in der ersten Stellung fixiert sind,
**Ermitteln** einer Öffnungsgröße (Q, V, X) des Luftdurchgangs (5) anhand des in der ersten Stellung erstellten dreidimensionalen Bilddatensatzes (11) des Luftdurchgangs (5),
**Kontrollieren** des Luftdurchganges (5) anhand des dreidimensionalen Bilddatensatzes (11), ob in der ersten Stellung die ermittelte Öffnungsgröße (Q, V, X) des Luftdurchgangs (5) einer Zielvorgabe entspricht,
für den Fall, dass die ermittelte Öffnungsgröße (Q, V, X) nicht der Zielvorgabe entspricht, Ermitteln eines Korrekturwertes anhand der Differenz der ermittelten Öffnungsgröße (Q, V, X) von der Zielvorgabe, wobei ausgehend von der ersten Stellung anhand des Korrekturwertes eine zweite Stellung definiert wird,
**Anfertigen** der Protrusionsschiene (6) derart, dass bei Auflage der Kieferbereiche (2, 3) an den jeweiligen Auflagebereichen (7, 8) der Unterkiefer (3) relativ zum Oberkiefer (2) in der zweiten Stellung gehalten ist.

2. Verfahren nach Anspruch 1,
**dadurch gekennzeichnet,**
**dass** der Korrekturwert anhand der Differenz der ermittelten Öffnungsgröße (Q, V, X) von der Zielvorgabe rechnerisch ermittelt wird, ohne dass es eines erneuten Erstellens eines dreidimensionalen Bilddatensatzes (11) des Luftdurchgangs (5) bedarf.

3. Verfahren nach Anspruch 1 oder 2,
**dadurch gekennzeichnet,**
**dass** für den Fall, dass die ermittelte Öffnungsgröße (Q, V, X) der Zielvorgabe nicht entspricht, die Schritte **Fixieren, Erstellen und Kontrollieren** nach Anspruch 1 vor den Schritten **Definieren** und **Anfertigen** erneut aber in einer geänderten ersten Stellung durchgeführt werden.

4. Verfahren nach einem der vorherigen Ansprüche,
**dadurch gekennzeichnet,**
**dass** die ermittelte Öffnungsgröße eine Querschnittsfläche (Q₁) des Luftdurchgangs (5), ein Volumen (V) des Luftdurchgangs (5) oder ein Abstand (X) zweier gegenüberliegender Wandungen des Luftdurchgangs (5) ist.

5. Verfahren nach einem der vorherigen Ansprüche,
**dadurch gekennzeichnet,**
**dass** die Kieferbereiche (2, 3) mithilfe einer verstellbaren Protrusionsschiene in der ersten Stellung **fixiert** werden.

6. Verfahren nach einem der vorherigen Ansprüche,
**dadurch gekennzeichnet,**
**dass** zur **Anfertigung** der Protrusionsschiene (6) die relative Ausrichtung zumindest eines Zahnes des Oberkieferbereichs (2) zu zumindest einem Zahn des Unterkieferbereichs (3) in der zweiten Stellung insbesondere mittels eines Bissregistrates ermittelt wird,
**dass** ein anhand eines Abbildes des Oberkieferbereichs und eines Abbildes des Unterkieferbereichs ein dreidimensionales Zahnnegativ (9) des Oberkieferbereichs (2) und ein dreidimensionales Zahnnegativ (10) des Unterkieferbereichs (3) angefertigt wird, und
**dass** die beiden Zahnnegative (9, 10) in der Protrusionsschiene (6) entsprechend der relativen Ausrichtung in zweiten Stellung zueinander angeordnet werden.

7. Verfahren nach einem der vorherigen Ansprüche,
**dadurch gekennzeichnet,**
**dass** zum **Anfertigen** der dreidimensionale Bilddatensatz (11) mit Oberflächendaten eines optischen Scans der Kieferbereiche (2, 3) überlagert wird, und dass daraus Zahnnegative (9, 10) berechnet werden, auf deren Basis die Protrusionsschiene (6) gefertigt wird.

8. Verfahren nach einem der vorherigen Ansprüche,
**dadurch gekennzeichnet,**
**dass** im Schritt **Erstellen** des dreidimensionalen Bilddatensatzes (11) neben den dreidimensionalen Bilddaten des Luftdurchganges (5) auch dreidimensionale Bilddaten der Kiefergelenke erstellt werden.

## Claims

1. A method for producing a protrusion splint (6),
wherein the protrusion splint (6) comprises an upper contact region (7) for defined contact for an upper jaw region (2) and a lower contact region (8) for defined contact for a lower jaw region (3), wherein the lower jaw region (3) is held in a position determined by the protrusion splint (6) relative to the upper jaw region (2) when the jaw regions (2, 3) contact the respective contact regions (7, 8), comprising the following method steps:
**Fixing** the jaw regions (2, 3) in a first position, wherein the lower jaw region (3) is shifted forwards relative to the upper jaw region (2) in the first position compared to the normal position of the jaws,
**Creating** a three-dimensional image dataset (11) of an air passage (5) in the region of the trachea (5) while the jaw regions (2, 3) are fixed in the first position,
**Determining** an opening size (Q, V, X) of the air passage (5) on the basis of the three-dimensional image dataset (11) of the air passage (5) created in the first position,
**Checking** the air passage (5) on the basis of the three-dimensional image dataset (11), as to whether the determined opening size (Q, V, X) of the air passage (5) in the first position corresponds to a target specification,
in the event that the determined opening size (Q, V, X) does not correspond to the target specification, determination of a correction value on the basis of the difference between the determined opening size (Q, V, X) and the target specification, wherein proceeding from the first position a second position is defined on the basis of the correction value,
**Producing** the protrusion splint (6) such that the lower jaw (3) is held in the second position relative to the upper jaw (2) when the jaw regions (2, 3) contact the respective contact regions (7, 8).

2. The method according to claim 1,
**characterised in that**
the correction value is determined by calculation on the basis of the difference between the determined opening size (Q, V, X) and the target specification, without a renewed creation of a three-dimensional image dataset (11) of the air passage (5) being required.

3. The method according to claim 1 or 2,
**characterised in that,**
in the event that the determined opening size (Q, V, X) does not correspond to the target specification, the steps of **fixing, creating and checking** according to claim 1 are carried out before the steps of **defining** and **producing** anew, but in a changed first position.

4. The method according to any one of the preceding claims,
**characterised in that**
the determined opening size is a cross-sectional (Q₁) of the air passage (5), a volume (V) of the air passage (5) or a distance (X) between two opposite walls of the air passage (5).

5. The method according to any one of the preceding claims,
**characterised in that**
the jaw regions (2, 3) are **fixed** in the first position with the aid of an adjustable protrusion splint.

6. The method according to any one of the preceding claims,
**characterised in that,**
for the **production** of the protrusion splint (6), the relative alignment of at least one tooth of the upper jaw region (2) to at least one tooth of the lower jaw region (3) is determined in the second position, in particular by means of a bite register,
a three-dimensional tooth negative (9) of the upper jaw region (2) and a three-dimensional tooth negative (10) of the lower jaw region (3) is produced on the basis of an image of the upper jaw region and an image of the lower jaw region, and
the two tooth negatives (9, 10) are arranged with respect to one another corresponding to the relative alignment in the second position in the protrusion splint (6).

7. The method according to any one of the preceding claims,
**characterised in that,**
for the **production,** the three-dimensional image dataset (11) is superimposed with surface data of an optical scan of the jaw regions (2, 3), and that tooth negatives (9, 10) are calculated therefrom, on the basis of which the protrusion splint (6) is produced.

8. The method according to any one of the preceding claims,
**characterised in that,**
in the step of **creating** the three-dimensional image dataset (11), three-dimensional image data of the jaw joints are created apart from the three-dimensional image data of the air passage (5).

## Revendications

1. Procédé de fabrication d'une gouttière de protrusion (6),
dans lequel la gouttière de protrusion (6) comprend une plage d'appui supérieure (7) en vue d'un appui défini pour une partie de la mâchoire supérieure (2) et une plage d'appui inférieure (8) en vue d'un appui défini pour une partie de mâchoire inférieure (3), dans lequel lors de l'appui des parties de mâchoire (2, 3) sur les parties d'appui (7, 8) respectives, la partie de mâchoire inférieure (3) est maintenue dans une position prédéfinie par la gouttière de protrusion (6) par rapport à la partie de mâchoire supérieure (2), comprenant les étapes de procédé suivant :
fixation des parties de mâchoire (2, 3) dans une première position, dans lequel dans la première position, la partie de mâchoire inférieure (3) est disposée vers l'avant par rapport à la partie de mâchoire supérieure (2), comparé à la position normale de la mâchoire,
création d'un jeu de données d'images tridimensionnelles (11) d'un passage d'air (5) au niveau de la trachée (5) tandis que les parties de mâchoire (2, 3) sont fixées dans la première position, calcul d'une grandeur d'ouverture (Q, V, X) du passage d'air (5) à l'aide du jeu de données d'images tridimensionnelles (11) du passage d'air (5) créé dans la première position,
contrôle du passage d'air (5) à l'aide du jeu de données d'images tridimensionnelles (11) pour voir si dans la première position, la grandeur d'ouverture (Q, V, X) calculée du passage d'air (5) correspond à un objectif,
dans le cas où la grandeur d'ouverture (Q, V, X) calculée ne correspond pas à l'objectif, calcul d'une valeur de correction à l'aide de la différence entre la grandeur d'ouverture (Q, V, X) et l'objectif, dans lequel en partant de la première position, une seconde position est calculée à l'aide de la valeur de correction,
fabrication de la gouttière de protrusion (6) de telle sorte que lors de l'appui des parties de mâchoire (2, 3) sur les parties d'appui (7, 8) respectives, la mâchoire intérieure (3) soit maintenue dans la seconde position par rapport à la mâchoire supérieure (2).

2. Procédé selon la revendication 1, **caractérisé en ce que** la valeur de correction est déterminée par calcul à l'aide de la différence entre la grandeur d'ouverture (Q, V, X) et l'objectif, sans que qu'il y ait besoin de créer de nouveau un jeu de données d'images tridimensionnelles (11) du passage d'air (5).

3. Procédé selon la revendication 1 ou 2, **caractérisé en ce que**
dans le cas où la grandeur d'ouverture (Q, V, X) calculée ne correspond pas à l'objectif, les étapes de fixation, création et contrôle selon la revendication 1 sont exécutées à nouveau mais dans une première position modifiée avant les étapes de définition et de fabrication.

4. Procédé selon l'une des revendications précédentes, **caractérisé en ce que**
la grandeur d'ouverture calculée est une surface transversale (Q₁) du passage d'air (5), un volume (V) du passage d'air (5) ou un écart (X) de deux parois opposées du passage d'air (5).

5. Procédé selon l'une des revendications précédentes, **caractérisé en ce que**
les parties de mâchoire (2, 3) sont fixées dans la première position à l'aide d'une gouttière de protrusion réglable.

6. Procédé selon l'une des revendications précédentes, **caractérisé en ce que**
pour fabriquer la gouttière de protrusion (6), l'alignement relatif d'au moins une dent de la mâchoire supérieure (2) par rapport à au moins une dent de la mâchoire inférieure (3) dans la seconde position est déterminé en particulier au moyen d'un enregistrement de morsure,
qu'avec une image de la mâchoire supérieure et une image de la mâchoire inférieure, un négatif (9) tridimensionnel des dents de la mâchoire supérieure (2) et un négatif (10) tridimensionnel des dents de la mâchoire inférieure (3) sont fabriqués et
que les deux négatifs de dents (9, 10) sont disposés entre eux dans la gouttière de protrusion (6) conformément à l'alignement relatif dans la seconde position.

7. Procédé selon l'une des revendications précédentes, **caractérisé en ce que**
pour la fabrication, le jeu de données d'images tridimensionnelles (11) est superposé avec des données de surface d'un scan optique des parties de mâchoire (2, 3) et que des négatifs de dents (9, 10) en sont calculés sur la base desquels la gouttière de protrusion (6) est fabriquée.

8. Procédé selon l'une des revendications précédentes, **caractérisé en ce qu'**à l'étape de création du jeu de données d'images tridimensionnelles (11), des données d'image tridimensionnelles de l'articulation de la mâchoire sont également créées outre les données d'image tridimensionnelles du passage d'air (5).
